# EUROPEAN PATENT APPLICATION

(11) **EP 1 466 923 A1**
(43) Date of publication of application: **13.10.2004**
(21) Application number: 04008008.7
(22) Date of filing: 01.04.2004
(51) Int. Cl.: C07K 14/79, A23C 9/146, A23J 1/20

(54) **Method for producing lactoferrin**

(30) Priority: 01.04.2003 JP 2003098597
(71) Applicant: SNOW BRAND MILK PRODUCTS CO., LTD., Sapporo, Hokkaido 065-0043 (JP)
(72) Inventor: Shigematsu, Akinori, Kawagoe-shi Saitama (JP); Sato, Kaoru, Kawagoe-shi Saitama (JP); Teduka, Nobuyuki, Kawagoe-shi Saitama (JP); Shiba, Mayumi, Tokorozawa-shi Saitama (JP); Tomizawa, Akira, Iruma-shi Saitama (JP)
(74) Representative: VOSSIUS & PARTNER

(57) **Abstract**

A method for producing lactoferrin, characterized in that lactoferrin is eluted into a solution with a salt content of 1 M or above from a hydrophilic, strong-acid cation-exchange resin to which lactoferrin has been adsorbed by allowing a concentrate of milk material produced by a filter membrane with a pore size of 0.05 µm to 0.2 µm to contact the cation-exchange resin.

## Description

The present invention relates to a method for separating and producing high-purity lactoferrin, which is a milk protein offering various bioactivation functions as well as important pharmacological properties, from milk that contains such lactoferrin.

Lactoferrin is a basic iron-binding protein present in milk and other external secretions and provides various bioactivation functions such as production of lymphocyte, promotion of iron absorption, normalization of leukocytes division, prevention of lipid peroxide production and demonstration of antiviral effect. For this reason, lactoferrin provides an important milk protein that can be used in drugs, animal feeds and food materials.

Accordingly, methods to separate and produce lactoferrin from milk have been studied in the past. Casein and whey protein, which are main proteins present in milk, have an isoelectric point (pI) of around 4.6 for casein and between 5.1 and 5.4 for whey protein, which are significantly different from lactoferrin's isoelectric point found near 9.5. The present mainstream technologies to separate lactoferrin focus on this unique isoelectric point of lactoferrin and mostly adopt methods to cause milk to contact a cation ion-exchanger at a pH value of 6 or above to let the ion-exchanger selectively adsorb positively charged lactoferrin and then separate it from other milk proteins that are charged with negative electricity at this pH level. However, cow milk also contains lactoperoxidase, which is a basic protein whose molecular weight and isoelectric point are very similar to those of lactoferrin. Since separation of lactoferrin and lactoperoxidase is difficult, separating high-purity lactoferrin from cow milk required a complex process and longer separation time and the lactoferrin recovery is also lower.

One commonly used lactoferrin production technology is the method to separate lactoferrin through affinity chromatography by fixing a substance having strong affinity with lactoferrin. For example, a ligand such as heparin (Japanese Patent Application Laid-open No. 63-255299), sulfuric ester compound (Japanese Patent Application Laid-open No. 63-255300) or sulfone group (Japanese Patent Application Laid-open No. 3-109400) is fixed to a hydrophilic carrier (cellulose, chitosan, silica, etc.) and the carrier is caused to contact milk material containing lactoferrin, thus letting lactoferrin adsorb to the carrier and then elute into a 0.5 to 1.0M sodium chloride solution.

Reported methods to separate/produce lactoferrin from milk in volumes suitable for industrial applications include the method to let skim milk or whey contact a weak-acid cation-exchange resin with a carboxymethyl group, flush and remove the non-adsorbed content with water, and elute step by step lactoperoxidase and lactoferrin into a 0.4 to 12% saline solution (Japanese Patent Application Laid-open No. 63-152400), and the method to let milk material contact a cation-exchange resin, flush with water, elute lactoperoxidase into a 0.35M sodium chloride solution, and then elute lactoferrin into a 1.0M sodium chloride solution (U.S. Patent Gazette for U.S. Patent No. 5596082).

These methods adopt a two-step elution process to increase the purity of lactoferrin, wherein a low-concentration saline solution is used to remove lactoperoxidase from a resin to which basic protein has been adsorbed, and then a high-concentration saline solution is used to elute/recover lactoferrin.

However, among the conventional lactoferrin separation/production methods mentioned above the separation method using affinity chromatography requires a large amount of ligand, which is difficult to obtain. This method also has other problems, such as the risk of fixed ligand dropping and mixing into lactoferrin, weak physical strength of carriers, and limitations regarding resin height in column and flow rate. For these reasons, application of this method for industrial use has been extremely difficult in actuality. Additionally, although they can yield high-purity lactoferrin with a lactoferrin content of 98% or above in protein, the methods adopting a two-step elution process also have disadvantages such as having to prepare multiple eluents and a long elution process that covers two elution sessions.

The present invention was developed in light of the above situations and aims to provide a method for efficient separation/production of high-purity lactoferrin in volumes suitable for industrial applications, through a single elution session by causing only lactoferrin, among all basic proteins, to be selectively adsorbed to resin.
According to the present invention this technical problem has been solved by the finding that by processing milk material containing lactoferrin through a filter membrane with a pore size of 0.05 µm to 0.2 µm to selectively filter lactoperoxidase into the filtrate, lactoferrin would be selectively concentrated into the concentrate.

With this filtration process, by processing the concentrate, into which lactoferrin has been selectively concentrated and which therefore has a higher concentration of lactoferrin than that of lactoperoxidase, through strong-acid cation-exchange resin, lactoferrin concentrates more selectively and the lactoferrin content in the eluent can be increased easily. These findings led to the present invention.

The basic method for producing lactoferrin as proposed by the present invention consists of three steps: (1) concentrate a solution containing lactoferrin using a filter membrane with a pore size of 0.05 µm to 0.2 µm and thereby increase the lactoferrin content in the concentrate, (2) cause the concentrate to contact hydrophilic strong-acid cation-exchange resin to let the resin selectively adsorb lactoferrin, and (3) elute adsorbed lactoferrin from the aforementioned strong-acid cation-exchange resin into a saline solution of 1.0 M or above.

The present invention allows any material for producing acid casein, rennet casein or various cheese to be used as the concentrate as obtained in process (1), and the generated whey as the loading material in process (2).

The basic method proposed by the present invention as described above produces pure lactoferrin of 90 weight-percent or higher in recovered protein. By adjusting the feed volume through the strong-acid cation-exchange resin and also by adjusting the electrical conductivity of the feed to an appropriate level, high-purity lactoferrin of 98 weight-percent or above can be obtained in recovered protein only through normal saline elution.

In addition, the present invention can keep the quality of milk material, partly because nothing is added to the material to separate lactoferrin in the aforementioned method. Since a concentrate of milk material produced by membrane filtration is used, the volume of feed to be loaded through the strong-acid cation-exchange resin can be reduced compared with the conventional methods, which shortens the loading time through the strong-acid cation-exchange resin.

Although the present invention permits use of various milk materials including milk and whey containing lactoferrin as derived from human, cow, buffalo, sheep, goat and other mammals, those with a gradual heat treatment curve are more desirable. It is because under high temperatures the properties of lactoferrin and lactoperoxidase, including membrane separation/adsorption characteristics and bioactivity, change significantly.

Ideally these milk materials should be filtered by bactofuse or using a microfiltration (MF) membrane with a pore size of 1.0 µm to 2.0 µm to remove precipitates and microorganisms beforehand. This pre-process increases the adsorption efficiency of protein to ion-exchanger.

A filter membrane with a pore size of 0.05 µm to 0.2 µm as shown in Test 1 is sufficient for use in the present invention to separate lactoperoxidase and lactoferrin in milk material. However, a MF membrane with a pore size of 0.1 µm is preferred in consideration of lactoferrin leak into the filtrate and of lactoperoxidase permeability. The concentration ratio via membrane filtration is normally two to five times. The percentages of lactoferrin and lactoperoxidase in protein change depending on this concentration ratio. In addition, since increasing the lactoferrin content among the basic proteins in the concentrate will increase the adsorption efficiency of lactoferrin to strong-acid cation-exchange resin and reduce the loading time, a concentration ratio of three times or higher is preferred. Better still, acid casein whey obtained when acid casein is prepared from a three-times or higher concentrate has an electrical conductivity of 13 mS/cm or above, which makes such acid casein whey an ideal milk material for producing high-purity lactoferrin for the reasons explained later.

Although the temperature for membrane filtration is not specified, the filtration temperature is normally 4°C or above but below 60°C. Temperatures below 4°C produce negative effects on the milk material, such as freezing, higher viscosity and separation of fat content. Temperatures above 60°C may cause lactoferrin and lactoperoxidase to change their properties.

The strong-acid cation-exchange resin used for adsorption of lactoferrin in the present invention may be produced by introducing a sulfone group into a hydrophilic matrix such as a bridged polysaccharide or hydrophilic vinyl polymer, or by introducing some kind of spacer between the resin and the exchange material. Specific examples include Chitopearl-SU (Fuji Spinning) containing a sulfone group, and SP Toyopearl (Tosoh) containing a sulfopropyl group.

Ways to cause milk material to contact strong-acid cation-exchange resin include the method to achieve contact inside a tank and the method that loads milk material through a column filled with strong-acid cation exchange resin. In particular, the column loading method provides an efficient way to prepare large volumes of high-purity lactoferrin. There are no specific temperatures at which milk material and strong-acid cation-exchange resin should be caused to contact each other. However, normally this process is performed at 4°C or above but below 60°C. Temperatures below 4°C produce negative effects on the milk material, such as freezing, higher viscosity and separation of fat content. Temperatures above 60°C may cause lactoferrin and lactoperoxidase to change their properties.

At 15°C or above, growth of microorganisms increases markedly. Therefore, the column loading temperature should be kept to below 15°C in industrial applications.

The volume of milk material caused to contact strong-acid cation-exchange resin is an important factor that determines the adsorption and purity of lactoferrin. The adsorption initially increases in proportion to the contact volume, but the rate of increase gradually decreases and finally reaches a saturation point. As the lactoferrin adsorption increases toward saturation, the lactoferrin purity also increases. This is because each basic protein has unique selectivity with respect to strong-acid cation-exchange resin. Lactoferrin replaces other basic proteins with low selectivity near its adsorption saturation point and adsorbs to the strong-acid cation-exchange resin.

Therefore, in order to obtain high-purity lactoferrin in an efficient manner, one needs to determine the minimum resin-contact volume in accordance with a specific combination of milk material and strong-acid cation-exchange resin. Since this minimum contact volume is inversely proportional to the concentration ratio in the membrane filtration process, use of milk material in a concentrated form is more desirable in view of production efficiency.

As a result of studying the relationship of selectivity of lactoferrin and lactoperoxidase with respect to strong-acid cation-exchange resin on one hand, and electrical conductivity of milk material on the other, it has been found, as shown in Test 2, that the selectivity of lactoferrin increases suddenly over that of lactoperoxidase when the electrical conductivity of milk material is 13 mS/cm or above. In other words, by adjusting the electrical conductivity of milk material to be processed to 13 mS/cm or above the effect of concentrating the material via membrane filtration is further augmented by a favorable change in selectivity, which further increases the purity of lactoferrin.

It has also been found that when acid casein is produced from a concentrate of skim milk filtered to a 1/3 or less of its original volume, generated acid casein whey exhibits an electrical conductivity of 13 mS/cm or above, and therefore a desired electrical conductivity can be obtained without having to adjust it by adding salt. Of course, this doesn't limit the milk material offering desired electrical conductivity to acid casein whey. Any milk material that has been filtered and exhibits an electrical conductivity of 13 mS/cm or above is sufficient.

The process proposed by the present invention allows for effective production of functional milk products without wasting milk material.

The present invention is explained in detail by using tests and examples.

### [Test 1]

Skim milk of pH 6.6 was concentrated to 1/3 of its original volume at 50°C using total five ceramic membranes including four types of ceramic membranes with pore sizes of 0.5, 0.2, 0.1 and 0.05 µm, respectively (Membralox) and a UF ceramic membrane with a molecular weight cutoff of 10 kD (equivalent to a pore size of less than 0.05 µm) (NGK), to obtain five membrane concentrates.

Then, 3.9 L of each of the above five membrane concentrates was loaded through five columns of 3 cm in inner diameter, each filled with 130 mL of SP-Toyopearl 550 (Tosoh) hydrophilic strong-acid cation-exchange resin, at a temperature of 10°C and flow rate of 1.0 L/h, after which 1.1 L of demineralized water was loaded through the columns at a flow rate of 1.6 L/h for cleaning, followed by another loading of 0.65 L of 9% saline solution at a flow rate of 0.8 L/h to elute the adsorbed content, to obtain 0.65 L each of five recovered solutions.

The obtained five recovered solutions were dialyzed using Spectra/Por 1 Membrane (Spectrum Laboratories), and then lactoferrin and lactoperoxidase concentrations were measured via FPLC using Resource S Column (Amersham Bioscience) at an absorbance of 280 nm.

Thereafter, lactoferrin purity was calculated from the peak area of lactoferrin relative to the total peak area on the FPLC chromatogram.
The results are shown in Table 1.

**[Table 1]**

| Pore size of filter | Less than | | | | |
|---|---|---|---|---|---|
| membrane [µm] | 0.05 | 0.05 | 0.1 | 0.2 | 0.5 |
| Lactoferrin [mg/L] | 3,300 | 3,330 | 3,240 | 2,550 | 1,450 |
| Lactoperoxidase [mg/L] | 680 | 370 | 310 | 280 | 140 |
| Lactoferrin purity [%] | 79 | 87 | 91 | 89 | 85 |

As shown in the table, the solution processed by a filter membrane with a pore size of 0.5 µm resulted in significantly less recovery of lactoferrin and lower purity. The solution processed by a UF ceramic membrane with a molecular weight cutoff of 10 kD, which corresponds to less than 0.05 µm in pore size, yielded higher lactoferrin recovery but a higher lactoperoxidase content reduced lactoferrin purity.

On the other hand, the solutions processed by filter membranes with pore sizes of 0.05, 0.1 and 0.2 µm offered satisfactory levels of both lactoferrin recovery and purity. In terms of recovery and purity, the solution processed by a filter membrane with a pore size of 0.1 µm offered the best results.

### [Example 1]

Skim milk of pH 6.6 was concentrated to 1/3 of its original volume at 50°C using a MF ceramic membrane with a pore size of 0.1 µm (Membralox) to obtain a MF-membrane concentrate of pH 6.7 with an electrical conductivity of 4.3 mS/cm.

Then, 21 L of the above MF-membrane concentrate was loaded through a column of 10 cm in inner diameter, filled with 700 mL of SP-Toyopearl 550 (Tosoh) hydrophilic strong-acid cation-exchange resin, at a temperature of 10°C and flow rate of 4.2 L/h, after which 5.6 L of demineralized water was loaded at a flow rate of 8 L/h for cleaning, followed by another loading of 3.5 L of 9% saline solution at a flow rate of 4.2 L/h to elute the adsorbed content, to obtain 3.5 L of recovered solution.

The MF-membrane concentrate passing through the column showed no particular changes in its pH, electrical conductivity, color, flavor, etc., compared with those before the loading process.

When the recovered solution was measured in the same manner as in Test 1, the lactoferrin and lactoperoxidase concentrations were 3,410 mg/L and 330 mg/L, respectively, and the lactoferrin purity was 90%.

### [Comparison Example 1]

Except that 63 L of unfiltered skim milk (pH 6.6, electrical conductivity 4.3 mS/cm) was used instead of the MF-membrane concentrate, the same procedure described in Example 1 was used to obtain 3.5 L of recovered solution.

The skim milk passing through the column showed no particular changes in its pH, electrical conductivity, color, flavor, etc., compared with those before the loading process.

When the recovered solution was measured in the same manner as in Test 1, the lactoferrin and lactoperoxidase concentrations were 3,420 mg/L and 580 mg/L, respectively, and the lactoferrin purity was 82%.

From the results of Example 1 and Comparison Example 1, it has been found that lactoferrin of higher purity can be obtained when skim milk concentrated by MF membrane is passed through hydrophilic strong-acid cation-exchange resin.

### [Example 2]

Skim milk of pH 6.6 was concentrated to 1/3.5 of its original volume at 50°C using a MF ceramic membrane with a pore size of 0.1 µm (Membralox) to obtain a MF-membrane concentrate of pH6.7 with an electrical conductivity of 4.3 mS/cm.

Then, 51 L of the above MF-membrane concentrates was loaded through a column of 10 cm in inner diameter, filled with 1,000 mL of SP-Toyopearl 550 (Tosoh) hydrophilic strong-acid cation-exchange resin, at a temperature of 10°C and flow rate of 4.2 L/h, after which 8 L of demineralized water was loaded at a flow rate of 8 L/h for cleaning, followed by another loading of 5 L of 9% saline solution at a flow rate of 4.2 L/h to elute the adsorbed content, to obtain 5 L of recovered solution.

The MF-membrane concentrate passing through the column showed no particular changes in its pH, electrical conductivity, color, flavor, etc., compared with those before the loading process.

When the recovered solution was measured in the same manner as in Test 1, the lactoferrin and lactoperoxidase concentrations were 5,340 mg/L and 340 mg/L, respectively, and the lactoferrin purity was 92%.

### [Comparison Example 2]

Except that 180 L of skim milk not processed by MF membrane (pH 6.6, electrical conductivity 4.3 mS/cm) was used instead of the MF-membrane concentrate, the same procedure described in Example 2 was used to obtain 5 L of recovered solution.

The skim milk passing through the column showed no particular changes in its pH, electrical conductivity, color, flavor, etc., compared with those before the loading process.

When the recovered solution was measured in the same manner as in Test 1, the lactoferrin and lactoperoxidase concentrations were 5,950 mg/L and 810 mg/L, respectively, and the lactoferrin purity was 87%.

From the results of Examples 1 and 2 and Comparison Example 2, it has been found that lactoferrin of higher purity can be obtained when the concentration rate of MF membrane filtering is increased, rather than when the lactoferrin adsorption is simply increased.

### [Test 2]

Skim milk of pH 6.6 was concentrated to 1/3.5 of its original volume at 50°C using a MF ceramic membrane with a pore size of 0.1 µm (Membralox), after which the concentrate was diluted to twice its original volume using demineralized water and then NaCl was added to adjust the electrical conductivity of the milk concentrate to 3, 6, 9, 13 and 16 mS/cm, to obtain five stock solutions.

Then, 10 L of each of the above five stock solutions was loaded through five columns of 3 cm in inner diameter, each filled with 130 mL of SP-Toyopearl 550 (Tosoh) hydrophilic strong-acid cation-exchange resin, at a temperature of 10°C and flow rate of 1.0 L/h, after which 1.1 L of demineralized water was loaded at a flow rate of 1.6 L/h for cleaning, followed by another loading of 0.65 L of 9% saline solution at a flow rate of 0.8 L/h to elute the adsorbed content, to obtain 0.65 L each of five recovered solutions.

The obtained five recovered solutions were measured in the same manner as in Test 1 for lactoferrin and lactoperoxidase concentrations as well as lactoferrin purity.
The results are shown in Table 2:

**[Table 2]**

| Electrical conductivity of stock solution [mS/cm] | 3 | 6 | 9 | 13 | 16 |
|---|---|---|---|---|---|
| Lactoferrin [mg/L] | 5,700 | 5,321 | 5,213 | 4,797 | 4,553 |
| Lactoperoxidase [mg/L] | 284 | 273 | 231 | 139 | 48 |
| Lactoferrin purity [%] | 93 | 93 | 94 | 96 | 98 |

As shown in the table, all recovered solutions provided satisfactory levels of both lactoferrin recovery and purity. However, it has been found that purity will become 95% or higher when the stock solution has an electrical conductivity of 13 mS/cm or above.

### [Example 3]

Skim milk of pH 6.6 was concentrated to 1/3.5 of its original volume at 50°C using a MF ceramic membrane with a pore size of 0.1 µm, after which the concentrate was cooled to 44°C and then hydrochloric acid was added to adjust the pH value to 4.5. Thus generated acid casein was separated to obtain whey.

Sodium hydroxide was added to the obtained whey to adjust its pH value to 6.8, after which the whey was centrifuged to remove precipitates to obtain whey of pH 6.7 with an electrical conductivity of 15 mS/cm.

The above whey was loaded through three columns (columns 1 through 3) of 3 cm in inner diameter, each filled with 130 mL of SP-Toyopearl 550 (Tosoh) hydrophilic strong-acid cation-exchange resin, at a temperature of 10°C and flow rate of 1.0 L/h, and in a volume of 4.2 L for column 1, 6.8 L for column 2 and 13.3 L for column 3. Thereafter, 1.1 L of demineralized water was loaded at a flow rate of 1.6 L/h for cleaning, followed by another loading of 0.65 L of 9% saline solution at a flow rate of 0.8 L/h to elute the adsorbed content, to obtain 0.65 L each of recovered solutions 1 through 3.

Each whey passing through the column showed no particular changes in its pH, electrical conductivity, color, flavor, etc., compared with those before the loading process.

When the recovered solutions were measured in the same manner as in Test 1, the lactoferrin and lactoperoxidase concentrations were 2,560 mg/L, 160 mg/L, respectively, and the lactoferrin purity was 93% for recovered solution 1. The values were 3,960 mg/L, 200 mg/L and 95% for recovered solution 2, and 7,350 mg/L, 50 mg/L and 98% for recovered solution 3.

### [Comparison Example 3]

Skim milk of pH 6.6 was heated to 44°C, hydrochloric acid was added to adjust its pH value to 4.5, and then the generated acid casein was separated to obtain whey.

Sodium hydroxide was added to the obtained whey to adjust its pH value to 6.8, after which the whey was centrifuged to remove precipitates to obtain whey of pH 6.7 with an electrical conductivity of 9 mS/cm.

Then, 18 L of the above whey was loaded through a column of 3 cm in inner diameter, filled with 130 mL of SP-Toyopearl 550 (Tosoh) hydrophilic strong-acid cation-exchange resin, at a temperature of 10°C and flow rate of 1.0 L/h. Thereafter, 1.1 L of demineralized water was loaded at a flow rate of 1.6 L/h for cleaning, followed by another loading of 0.65 L of 9% saline solution at a flow rate of 0.8 L/h to elute the adsorbed content, to obtain 0.65 L of recovered solution. The whey passing through the column showed no particular changes in its pH, electrical conductivity, color, flavor, etc., compared with those before the loading process.

When the recovered solution was measured in the same manner as in Test 1, the lactoferrin and lactoperoxidase concentrations were 2,440 mg/L, 620 mg/L, respectively, and the lactoferrin purity was 78%.

From the results of Example 3 and Comparison Example 3, it has been found that lactoferrin of high purity can be obtained by passing through hydrophilic strong-acid cation-exchange resin a whey obtained by preparing acid casein from a skim milk concentrate produced by MF membrane and then removing the acid casein, and that lactoferrin of even higher purity can be obtained by increasing the volume of milk material contacting the hydrophilic strong-acid cation-exchange resin.

It has been shown that the method proposed by the present invention provides lactoferrin of high purity by passing through hydrophilic strong-acid cation-exchange resin a skim milk concentrate produced via membrane filtration or a whey obtained by removing casein from the concentrate, and that lactoferrin of even higher purity can be obtained by increasing the volume of milk material contacting the hydrophilic strong-acid cation-exchange resin.

By using the method proposed by the present invention it is also possible to obtain, only through normal saline elution, high-purity lactoferrin of 98 weight-percent or higher in recovered protein by using material of an appropriate electrical conductivity and also adjusting the volume of material loaded through the hydrophilic strong-acid cation-exchange resin.

In addition, the separation method proposed by the present invention provides the advantage of being able to keep the quality of milk material, because nothing is added to milk material to separate lactoferrin. Furthermore, use of milk material concentrated by membrane filtration reduces the volume of material loaded through the strong-acid cation-exchange resin compared with the conventional methods, which shortens the loading time through the strong-acid cation-exchange resin.

The lactoferrin obtained by the present invention is a basic iron-binding protein present in milk and other external secretions and offers various bioactivation functions such as production of lymphocyte, promotion of iron absorption, normalization of leukocytes division, prevention of lipid peroxide production and demonstration of antiviral effect. For this reason, the lactoferrin obtained by the present invention provides an important milk protein that can be used in drugs, animal feeds and food materials.

## Claims

1. A method for producing lactoferrin, **characterized in that** lactoferrin is eluted into a solution with a salt content of 1 M or above from a hydrophilic, strong-acid cation-exchange resin to which lactoferrin has been adsorbed by allowing a concentrate of milk material produced by a filter membrane with a pore size of 0.05 µm to 0.2 µm to contact said cation-exchange resin.

2. The method as described in Claim 1, wherein said milk material is skim milk and/or whey containing lactoferrin.

3. A method for producing lactoferrin, **characterized in that** lactoferrin is eluted into a solution with a salt content of 1 M or above from a hydrophilic, strong-acid cation-exchange resin to which lactoferrin has been adsorbed by allowing a solution obtained by removing casein content from a skim milk concentrate produced by a filter membrane with a pore size of 0.05 µm to 0.2 µm to contact said cation-exchange resin.

4. The method as described in Claim 3, wherein said solution obtained by removing the casein content from the skim milk concentrate is whey generated when acid casein, rennet casein or various cheese is prepared from said skim milk concentrate.

5. The method as described in any one of Claims 1 to 4, wherein lactoferrin is adsorbed by adjusting the electrical conductivity of the solution contacting the strong-acid cation-exchange resin to 13 mS/cm or above.
